# EUROPEAN PATENT APPLICATION

(11) **EP 2 453 236 A2**
(43) Date of publication of application: **16.05.2012**
(21) Application number: 12154982.8
(22) Date of filing: 16.02.2007
(51) Int. Cl.: G01N 33/53, G01N 33/68

(54) **Biological load indicator and method of measuring biological load**

(30) Priority: 17.02.2006 JP 2006041633
(62) Divisional of application: 07714417.8
(71) Applicant: Sekiyama, Atsuo, Suita-shi, Osaka 5640063 (JP)
(72) Inventor: Sekiyama, Atsuo, Suita-shi, Osaka 5640063 (JP)
(74) Representative: Duckworth, Timothy John

(57) **Abstract**

Disclosed are techniques capable of objectively and specifically evaluating various mental or physical conditions, of which evaluation was conventionally possible only by subjective symptom-dependent methods, such as stress and fatigue. Specifically disclosed are a stress or fatigue indicating agent including at least two factors selected from the group consisting of IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF; an agent for testing stress or fatigue including at least two molecules selected from the group consisting of molecules specifically recognizing the factors, respectively; a method of measuring stress or fatigue with the test agent; and an indicating agent for evaluating the intensity of mental conditions or disorders, including at least two factors selected from the factor group, wherein each of the factors is weighted.

## Description

### Technical Field

The present invention relates to biological load indicators and methods for measuring biological loads. Specifically, the present invention relates to the technical field for objectively evaluating biological loads such as stress and fatigue by molecular-biological approaches.

### Background Art

Mental stress and/or physical stress affects host defense mechanisms including nervous, endocrine and immune systems (see non-patent references 1 and 2). Various cytokines (such as IL-1β, IL-6, and TNF-α) are upregulated by stress (see non-patent references 1 and 3). This suggests that cytokines are likely to be involved in interference with host defense (see non-patent reference 4). However, the molecular mechanism of the induction of host defense-reducing cytokines by stress is not fully understood.

Interleukin-18 (IL-18) is a cytokine that was discovered as an interferon-γ (IFN-γ)-inducing factor (see non-patent reference 5, patent reference 1). IL-18 has various biological activities such as Fas ligand induction, elevation of the cytolytic activity of T cells (see non-patent reference 6), and production of IL-4 and IL-13 (see non-patent reference 7). IL-18 activates Toll-like receptor 2 (see non-patent reference 9) and myeloid differentiation protein (Myd)-88 (see non-patent reference 10). These activations are necessary for IL-6 induction (see non-patent reference 11). Therefore, IL-18 is involved in the production of both Th1 and Th2 cytokines (see non-patent reference 12).

IL-18 is produced as a 24 kD precursor protein that is processed to a 18 kD mature active form by an IL-1β converting enzyme (ICE, also called caspase-1) (see non-patent reference 13). Caspase-1 is induced as an inactive precursor protein procaspase-1 that is activated by caspase-11 (see non-patent reference 14). It is reported that caspase-11 mRNA expression involves trans-activation of NF-κB (see non-patent reference 15), which is mediated by P38MAP kinase (see non-patent reference 16). It is also reported that the induction of caspase-11 mRNA by LPS (lipopolysaccharide) and the following activation of caspase-1 are inhibited by SB203580, a P38MAP kinase inhibitor, in a glioma cell line C6 (see non-patent reference 17)..

Recent studies report that IL-18 mRNA is expressed in an adrenal gland in response to an adrenocorticotropic hormone (ACTH) and cold stress (see non-patent reference 18). It is also reported that different promoters are used for IL-18 mRNA expression in an adrenal gland and an immune cell (see non-patent reference 19). However, both studies described above are silent on the induction of mature IL-18. On the other hand, it is reported that IL-18 in blood plasma increases in psychiatric patients (see non-patent reference 20).

In society today, people work and live under various types of stress. In general, there are differences in sensory perception of stress among individuals, and there is no specific indicator of the presence or absence of stress or the intensity of stress. As a result of investigations, the inventor has found that stress causes an increase in cytokines such as IL-18 and have revealed the flow of signal transduction with IL-18 at the top of the stress cascade, so that stress can be objectively evaluated (see patent reference 2, non-patent references 21 - 23).
patent reference 1: JP-A-8-193098
patent reference 2: W02006/003927
non-patent reference 1: Dugue, B. et al. Scand. J. Clin. Invest. 53, 555-561 (1993)
non-patent reference 2: Kiecolt-Glaser, J. K. et al. Proc. Natl. Acad. Sci. USA 93, 3043-3047 (1996)
non-patent reference 3: Endocrinology 133, 2523-2530 (1993) non-patent reference 4: Schubert, C. et al. Psychosom. Med. 61, 876-882 (1999)
non-patent reference 5: Zhou, D. et al. Nature 378, 88-91 (1995)
non-patent reference 6: Nakanishi, K. et al. Annu. Rev. Immunol. 19 423-474 (2001)
non-patent reference 7: Hoshino, T. et al. J. Immunol. 162, 5070-5077 (1999)
non-patent reference 8: Dinarello, C.A. et al. J. Leukoc. Biol. 63, 658-664 (1998)
non-patent reference 9: Blease, K. et al. Inflamm. Res. 50, 552-560 (2001)
non-patent reference 10: Adachi, O. et al. Immunity 9, 143-150 (1998)
non-patent reference 11: Takeuchi, O. et al. J. Immunol. 165, 5392-5396 (2000)
non-patent reference 12: Hoshino, T. et al. J. Immunol. 166, 7014-7018 (2001)
non-patent reference 13: Gu, Y. et al. Science 275, 206-209 (1997)
non-patent reference 14: Wang, S. et al. Cell 92, 501-509 (1998)
non-patent reference 15: Schauvliege, R. et al. J. Biol. Chem. 277, 41624-41630 (2002)
non-patent reference 16: Vanden Berghe, W. et al. J. Biol. Chem. 273, 3285-3290 (1998)
non-patent reference 17: Hur, J. et al. FEBS Lett. 507, 157-162 (2001)
non-patent reference 18: Conti, B. et al. J. Biol. Chem. 272, 2035-2037 (1997)
non-patent reference 19: Sugama, S. et al. J. Immunol. 165, 6287-6292 (2000)
non-patent reference 20: Kokai, M. et al. J. Immunother. 25, 68-71 (2002)
non-patent reference 21: Sekiyama, A. et al. Immunity 22(6), 669-677 (2005)
non-patent reference 22: Sekiyama, A. et al. J Neuroimmunol. 171(1-2), 38-44 (2006)
non-patent reference 23: Sekiyama, A. et al. J Med Invest. 52, 236-239 (2005)

### Disclosure of the Invention

### Problems to be Solved by the Invention

A living organism has a mechanism to keep its biological functions constant in response to loads and stimuli. Such a mechanism is called homeostasis or host-defense mechanism. It is known that mental, physiological, physical, or chemical stress and/or fatigue acts as a load on homeostasis or host-defense mechanism and that various diseases are caused by breakdown of homeostasis or host-defense mechanism.
A load on homeostasis or host-defense mechanism, such as stress, fatigue and blues can further cause mental disorders, physical disorders, and even suicide, and is a serious challenge to health. However, evaluation of such conditions basically accompanied by subjective symptoms has been possible only by self-assessment. It has also been very difficult to detect abnormalities by known biochemical or psychological tests, and therefore, it has been impossible to make objective evaluations, grasp severity or develop some remedies. Various biochemical or physiological indicators have been proposed. However, hormones or amines are instable and difficult to be quantified, and therefore, they are not suitable as indicators, although their level can change in conjunction with stress.
An object of the present invention is to provide techniques capable of objectively and specifically evaluating various loads on living organisms, of which evaluation was conventionally possible only by subjective symptom-dependent methods, such as fatigue. Since living organisms feel discomfort when they recognize loads on biological homeostasis and host-defense mechanism, the object of the present invention necessarily encompasses to provide techniques capable of objectively and specifically evaluating a feeling of discomfort or comfort in living organisms.

### Means for Solving the Problems

In light of the problems described above, the inventor has made active investigations and has found that an exhaustive survey of variations in the expression of cytokines or chemokines constituting an IL-18-centered biological cascade allows objective understanding of various biological loads such as stress and fatigue, so that the invention has been completed.
Accordingly, the present invention provides the following.

[1] An agent for indicating stress, comprising at least two kinds of factors selected from the group consisting of IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF.
[2] An agent for testing stress, comprising at least two kinds of molecules selected from the group consisting of molecules specifically recognizing IL-β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, respectively.
[3] An agent for indicating fatigue, comprising at least two kinds of factors selected from the group consisting of IL-β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, FGF-β, neurotrophin 5, MCP-3, β-2-microglobulin, angiotensin II, CSF-3, CXC chemokine ligand 1, CXC chemokine ligand 5 and HGF.
[4] An agent for testing fatigue comprising at least two kinds of molecules selected from the group consisting of molecules specifically recognizing IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, respectively.
[5] The test agent of the above-mentioned [2] or [4], wherein the molecule is an antibody.
[6] A method of measuring stress, comprising measuring the level of factors in a biological sample using the test agent of the above-mentioned [2] or [5].
[7] A method of measuring fatigue, comprising measuring the level of factors in a biological sample using the test agent of the above-mentioned [4] or [5].
[8] The method of the above-mentioned [6] or [7], wherein the above-mentioned biological sample is plasma, serum, saliva or urine.
[9] An indicating agent for evaluating mental conditions selected from the group consisting of mental fatigue, physical fatigue, stress, blue mood, comfortable mood, uncomfortable mood, dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk, which comprises at least two kinds of factors selected from the group consisting of IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF at a weighted composition ratio.
[10] An indicating agent for evaluating intensity of mental disorders selected from the group consisting of mental fatigue, stress, depression, depressive state, mood disorders, schizophrenia, obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt, which comprises at least two kinds of factors selected from the group consisting of IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF at a weighted composition ratio.
[11] An agent for testing mental conditions selected from the group consisting of mental fatigue, physical fatigue, stress, blue mood, comfortable mood, uncomfortable mood, dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk, which comprises at least two kinds of molecules selected from the group consisting of molecules specifically recognizing IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, respectively.
[12] An agent for testing intensity of mental disorders selected from the group consisting of mental fatigue, stress, depression, depressive state, mood disorders, schizophrenia, obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt, which comprises at least two kinds of molecules selected from the group consisting of molecules specifically recognizing IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, respectively.
[13] The test agent of the above-mentioned [11] or [12], wherein the aforementioned molecule is an antibody.
[14] A method of measuring mental conditions selected from the group consisting of mental fatigue, physical fatigue, stress, blue mood, comfortable mood, uncomfortable mood, dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk, which comprises a step of measuring the level of the factors in a biological sample using the test agent of the above-mentioned [11] or [13], and
   a step of comparing the amount obtained in the aforementioned measurement step with that of the indicating agent of the above-mentioned [9] by proportional weighting.
[15] A method of measuring the intensity of mental disorders selected from the group consisting of mental fatigue, stress, depression, depressive state, mood disorders, schizophrenia, obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt, which comprises a step of measuring the level of the factors in a biological sample using the test agent of the above-mentioned [12] or [13], and a step of comparing the amount obtained in the aforementioned measurement step with that of the indicating agent of the above-mentioned [10] by proportional weighting.
[16] The method of the above-mentioned [14] or [15], wherein the aforementioned biological sample is plasma, serum, saliva or urine.
[17] A kit for testing mental conditions selected from the group consisting of mental fatigue, physical fatigue, stress, blue mood, comfortable mood, uncomfortable mood, dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk, comprising, in separate compartments, at least two kinds of molecules selected from the group consisting of molecules specifically recognizing IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, respectively.
[18] A kit for testing the intensity of mental disorders selected from the group consisting of mental fatigue, stress, depression, depressive state, mood disorders, schizophrenia, obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt, comprising, in separate compartments, at least two kinds of molecules selected from the group consisting of molecules specifically recognizing IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, respectively.
[19] The kit of the above-mentioned [17] or [18], wherein the aforementioned molecule is an antibody.

### Effects of the Invention

The stress or fatigue indicating agent of the present invention including a group of factors having various in vivo effects, such as cytokines, allows objective and molecular-biological evaluation of the degree of stress or fatigue in a mammal such as human. The stress or fatigue test agent of the present invention including molecules capable of recognizing factors having various in vivo effects, such as cytokines, allows an objective and quantitative test of the degree of stress or fatigue in a mammal such as human. The stress or fatigue measurement method of the invention including measuring the concentration of factors in a biological sample with the test agent of the invention allows objective and quantitative measurement of the degree of stress or fatigue in a mammal such as human. The indicating agent of the present invention including the factors each of which is weighted allows objective, exhaustive and molecular-biological evaluation of the intensity of various mental conditions or disorders in a mammal such as human. The agent of the present invention for testing the intensity of mental conditions or disorders, which includes molecules capable of recognizing factors having various in vivo effects, such as cytokines, wherein each of the molecules is weighted, allows objective and quantitative test of the intensity of various mental conditions or disorders in a mammal such as human. The method of the present invention for measuring the intensity of mental conditions or disorders, which includes using the agent of the present invention for testing mental conditions or disorders and using the mental condition or disorder indicating agent of the present invention, allows objective and quantitative evaluation of the intensity of various mental conditions or disorders in a mammal such as human. The kit of the present invention for testing the intensity of mental conditions or disorders, which includes test agents of the present invention placed in different compartments, may be appropriately used for a single or exhaustive test of the intensity of the mental conditions or disorders.

Therefore, the present invention is useful for reconstruction of the basis of preventive medicine and public health administration, evaluation and control of life activity level and mental health of patients under medial treatment, evaluation and control of life activity level and mental health of convalescent patients, determination of the range of industrial or occupational health or work-related injuries and illness, evaluation of working environments, evaluation of working intensity, assessment of living or working environments, evaluation of school sanitation or learning environments or intensity, environmental assessment, evaluation of diseases or disorders mainly characterized by stress or fatigue (psychosomatic disorder, depression, psychoneurosis, chronic fatigue syndrome, personality disorder, character disorder, maladjustment syndrome, social withdrawal, burnout syndrome, apathy, psychogenic reactions, PTSD, and almost all other diseases), international unification of stress evaluation criteria, and establishment of international criteria for evaluation of each of the above.

### Brief Description of the Drawings

[Fig. 1a] Fig. 1a is a graph showing a correlation pattern of levels of blood plasma cytokines or chemokines in a total of 402 healthy subjects (with correlation coefficients of 0.50 to 0.6499 in light gray, 0.65 to 0.7999 in dark gray, 0.8 to 0.9999 in black; concerning the relative risk of correlation, p < 0.001 when the correlation coefficient is 0.50; in the table, darker shading indicates higher correlation, and the same applies to the other correlation tables described below).
[Fig. 1b] Fig. 1b is a graph showing a correlation pattern of levels of pre-night shift blood plasma cytokines or chemokines in 142 subjects who are healthy but frequently work night shifts.
[Fig. 1c] Fig. 1c is a graph showing a correlation pattern of post-night shift blood plasma cytokines or chemokines in the subjects.
[Fig. 1d] Fig. 1d is a classification table obtained by a logistic regression analysis to determine whether it is possible to correctly classify 90 people randomly sampled from the subjects into a pre-night shift group and a post-night shift group according to the present invention, in which the accuracy is 88% according to the invention.
[Fig. 2a] Fig. 2a is a graph showing a correlation pattern of levels of blood plasma cytokines or chemokines after a 1 hour of Kraepelin load (with correlation coefficients of 0.50 to 0.6499 in light gray, 0.65 to 0.7999 in dark gray, 0.8 to 0.9999 in black; concerning the relative risk of correlation, p < 0.001 when the correlation coefficient is 0.50; in the table, darker shading indicates higher correlation, and the same applies to the other correlation tables described below).
[Fig. 2b] Fig. 2b is a graph showing a correlation pattern of levels of blood plasma cytokines or chemokines after a 3 hours of Kraepelin load.
[Fig. 2c] Fig. 2c is a graph showing a correlation pattern of levels of blood plasma cytokines or chemokines after a 3 hours of Kraepelin load and a 3 hour rest after the load.
[Fig. 2d] Fig. 2d is a classification table obtained by a logistic regression analysis of the data on the 1 hour of Kraepelin load and the 3 hours of Kraepelin load.
[Fig. 2e] Fig. 2e is a classification table obtained by the same analysis of the data on the 3 hours of Kraepelin load and the data after the 3 hour rest after the load.
[Fig. 2f] Fig. 2f is a graph showing a correlation pattern of levels of blood plasma cytokines or chemokines after a 1 hour of treadmill exercise.
[Fig. 2g] Fig. 2g is a graph showing a correlation pattern of levels of blood plasma cytokines or chemokines after a 3 hours of treadmill exercise.
[Fig. 2h] Fig. 2h is a graph showing a correlation pattern of levels of blood plasma cytokines or chemokines after a 3 hours of treadmill exercise and a 3 hour rest after the exercise.
[Fig. 2i] Fig. 2i is a classification table obtained by a logistic regression analysis of the data before the treadmill exercise and the data after the 1 hour of treadmill exercise.
[Fig. 2j] Fig. 2j is a classification table obtained by the same analysis of the data after the treadmill exercise and the data on the next day.
[Fig. 2k] Fig. 2k is a classification table obtained by the same analysis of the data on the 1 hour of Kraepelin load and the data on the 1 hour of treadmill exercise.
[Fig. 21] Fig. 21 is a classification table obtained by the same analysis of the data on the 3 hours of Kraepelin load and the data on the 3 hours of treadmill exercise.

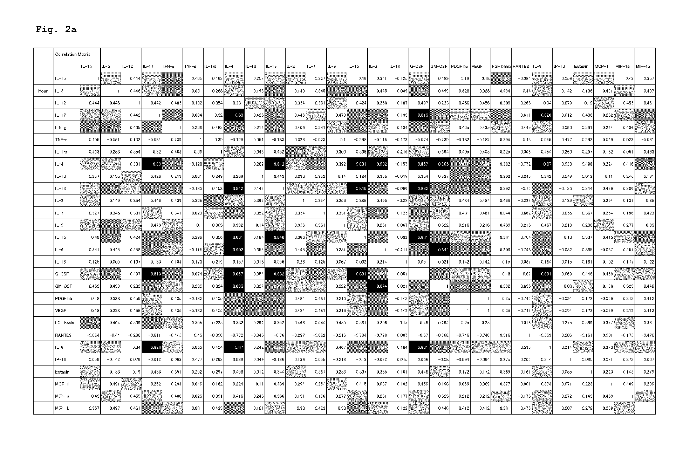

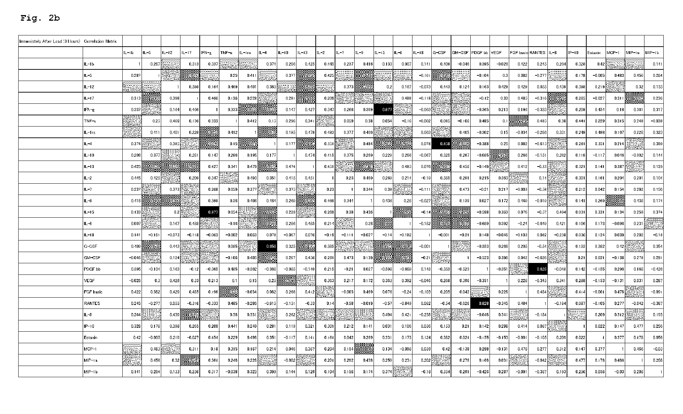

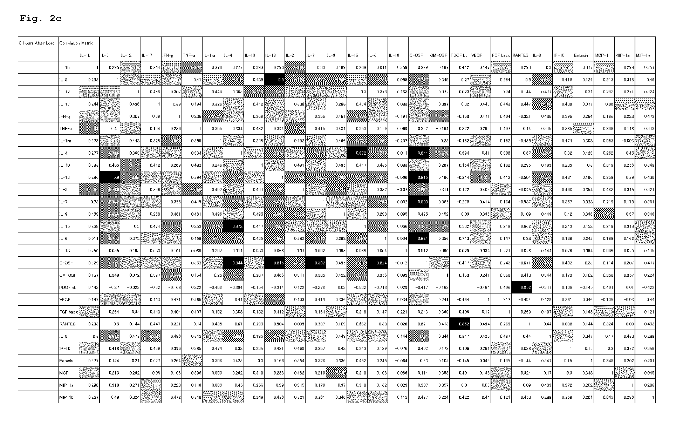

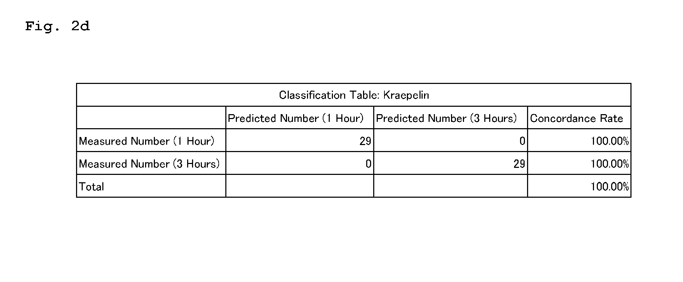

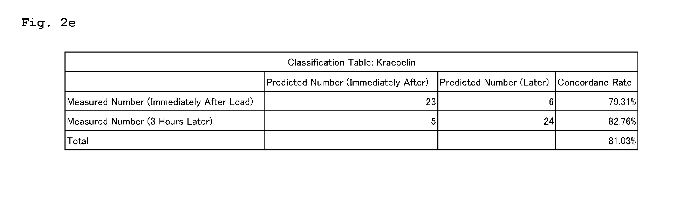

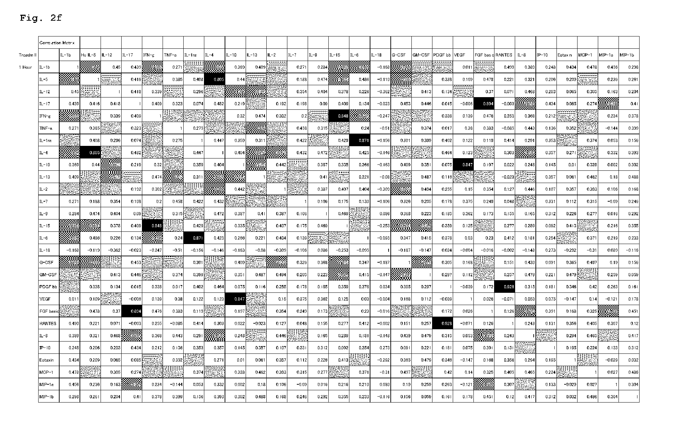

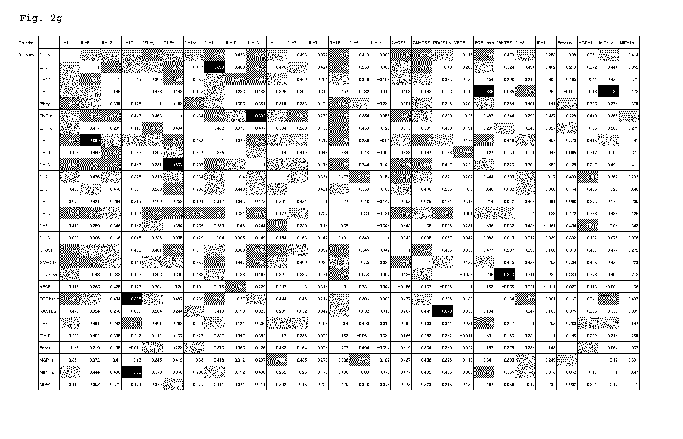

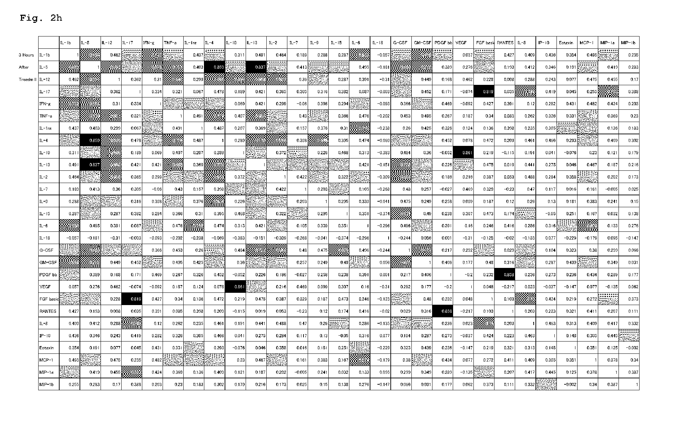

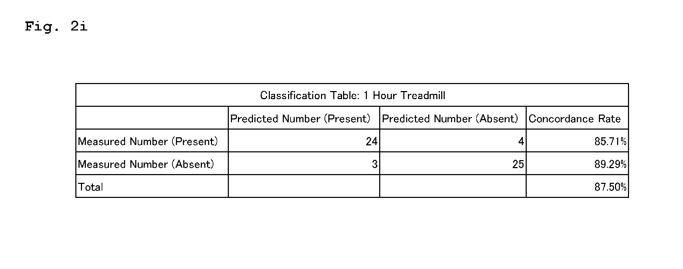

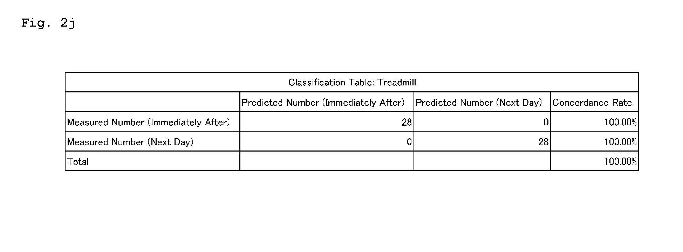

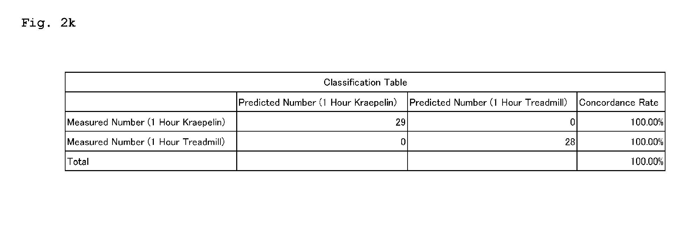

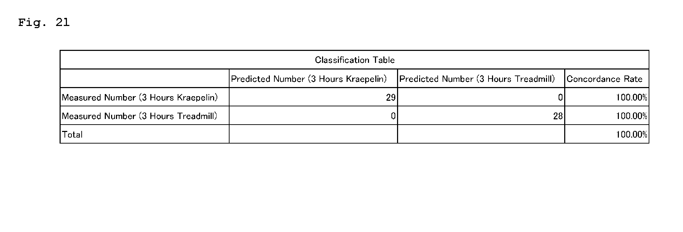

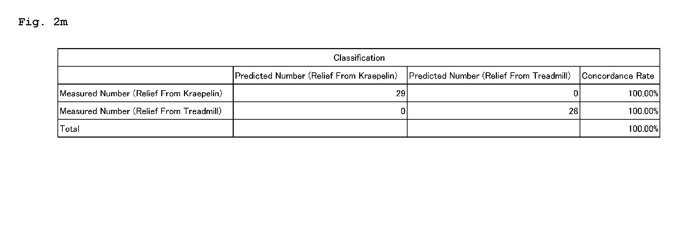

[Fig. 2m] Fig. 2m is a classification table obtained by the same analysis of the data of 3 hours of Kraepelin load followed by 3 hours of rest and the data of 3 hours of treadmill exercise followed by 3 hours of rest.

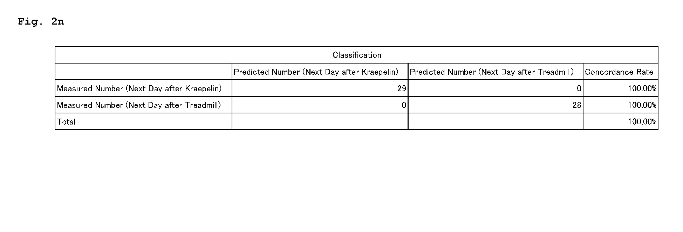

[Fig. 2n] Fig. 2n is a classification table between the group on the next day after the Kraepelin load and the group on the next day after the treadmill exercise.

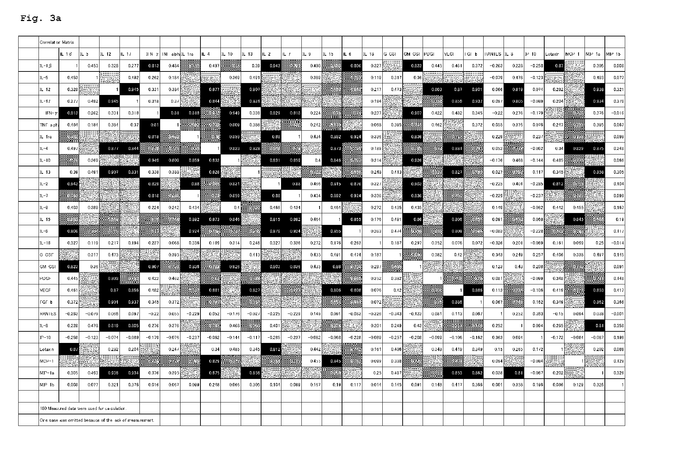

[Fig. 3a] Fig. 3a is a graph showing a correlation pattern of levels of blood plasma cytokines or chemokines in patients with depression (with correlation coefficients of 0.50 to 0.6499 in light gray, 0.65 to 0.7999 in dark gray, 0.8 to 0.9999 in black; concerning the relative risk of correlation, p < 0.001 when the correlation coefficient is 0.50; in the table, darker shading indicates higher correlation).

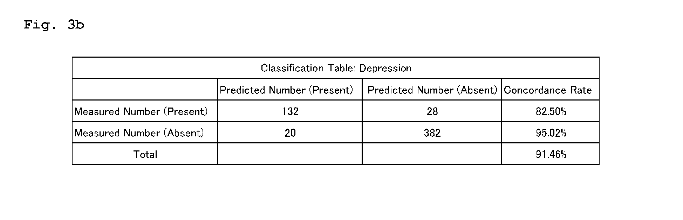

[Fig. 3b] Fig. 3b is a classification table obtained by a logistic regression analysis to determine whether it is possible to correctly classify the subjects into a healthy group and a depression patient group according to the present invention.

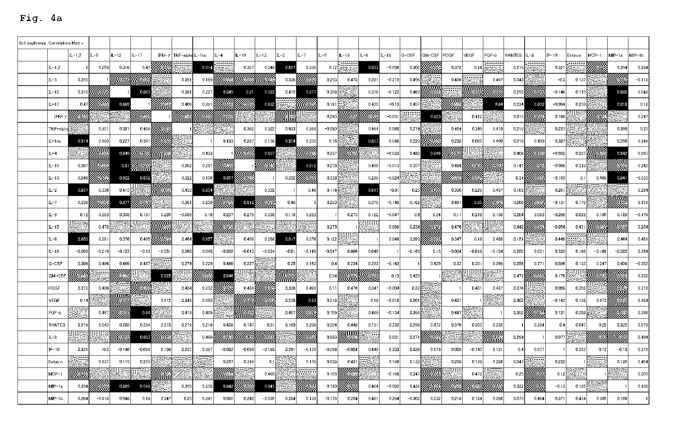

[Fig. 4a] Fig. 4a is a graph showing a correlation pattern of levels of blood plasma cytokines or chemokines in patients with schizophrenia (with correlation coefficients of 0.50 to 0.6499 in light gray, 0.65 to 0.7999 in dark gray, 0.8 to 0.9999 in black; concerning the relative risk of correlation, p < 0.001 when the correlation coefficient is 0.50; in the table, darker shading indicates higher correlation).

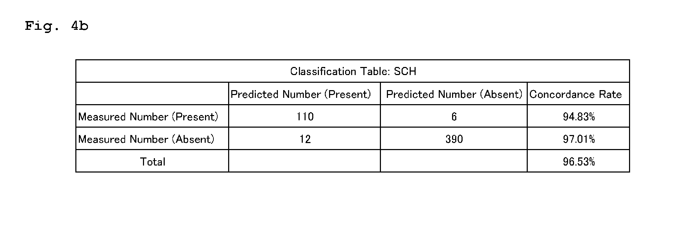

[Fig. 4b] Fig. 4b is a classification table obtained by a logistic regression analysis to determine whether it is possible to correctly classify the subjects into a healthy group and a schizophrenia patient group according to the present invention.

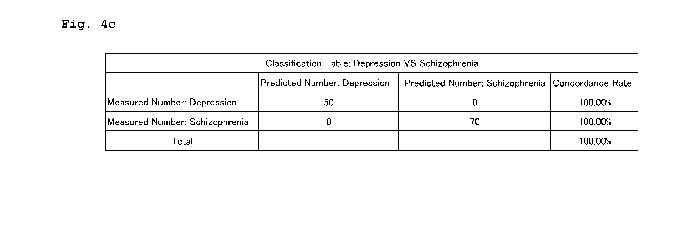

[Fig. 4c] Fig. 4c is a classification table obtained by the analysis to determine whether it is possible to correctly classify the subjects into a depression patient group and a schizophrenia patient group according to the present invention.

### Best Mode for Carrying Out the Invention

In the present invention, the characteristics to be evaluated may be classified into the state of loads in a living organism and the pathological condition of a living organism. In the present invention, the evaluation or determination level is, in the case of a state, the state itself and the degree of manifestation of the state, and in the case of a pathological condition, the type and degree of the manifested pathological condition. Concerning pathological conditions which manifest themselves but for which no objective evaluation criterion has been conventionally found, the present invention also aims to provide criteria for evaluating such pathological conditions through the approach of monitoring potential conditions, specifically, variations in cytokines or chemokines in a living organism.

The indicating agent of stress or fatigue of the present invention contains at least two kinds of factors selected from the group consisting of IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF.

As used herein, the term "indicating agent" refers to any in vivo marker that serves to objectively indicate various mental conditions, for which only self-assessment evaluation approaches have conventionally been established. The "indicating agent" may also be an in vivo marker that serves to objectively indicate the intensity of mental disorders.

As used herein, the term "biological load" means a chemical, physical, mental, verbal, or laborious load on the mental or physical condition of a living organism. The "biological load" is intended to include any endogenous constant load caused by pathological conditions or the like in a living organism. In the present invention, for example, the biological load also includes the breakdown of biological homeostasis, loading of the breakdown of biological homeostasis, the precursor state of the breakdown of biological homeostasis, the breakdown of host-defense mechanism, loading of the breakdown of host-defense mechanism, a precursor state of the breakdown of host-defense mechanism and the like.

As used herein, the term "stress" means various biological responses caused by the application of a chemical, physical, mental, verbal, or laborious temporary load to the mental or physical condition of a living organism. The "stress" also means various biological responses caused by the application of any endogenous constant load in a living organism.

As used herein, the term "fatigue" means any type of fatigue including physical fatigue and mental fatigue.

The factors contained in the indicating agent of the present invention are at least two kinds selected from the above-mentioned group. To provide a more specific index of fatigue, they are 3 to 41 kinds, preferably 3 to 28 kinds, more preferably 5 to 20 kinds, and still more preferably 8 to 12 kinds.

In the present invention, "IL-1β" is a material of which the human amino acid sequence and base sequence are published under the Genbank Accession No. NM_000576 or the like and which may be isolated or produced by publicly known methods. The "IL-1β" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-1 ra" is a material of which the human amino acid sequence and base sequence are published under the Genbank Accession No. NM_173841, NM_173842, NM_173843, NM_000577 or the like and which may be isolated or produced by publicly known methods. The "IL-1 ra" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-2" is a material of which the human amino acid sequence and base sequence are published under the Genbank Accession No. NM_000586 or the like and which may be isolated or produced by publicly known methods. The "IL-2" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-4" is a material of which the human amino acid sequence and base sequence are published under the Genbank Accession No. NM_000589, NM_172348 or the like and which may be isolated or produced by publicly known methods. The "IL-4" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-5" is a material of which the human amino acid sequence and base sequence are published under the Genbank Accession No. NM_000879 or the like and which may be isolated or produced by publicly known methods. The "IL-5" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-6" is a material of which the human amino acid sequence and base sequence are published under the Genbank Accession No. NM_000600 or the like and which may be isolated or produced by publicly known methods. The "IL-6" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-7" is a material of which the human amino acid sequence and base sequence are published under the Genbank Accession No. NM_000880 or the like and which may be isolated or produced by publicly known methods. The "IL-7" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-8" is a material of which the human amino acid sequence and base sequence are published under the Genbank Accession No. NM_000584 or the like and which may be isolated or produced by publicly known methods. The "IL-8" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-9" is a material of which the human amino acid sequence and base sequence are published under the Genbank Accession No. NM_000590 or the like and which may be isolated or produced by publicly known methods. The "IL-9" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-10" is a material of which the human amino acid sequence and base sequence are published under the Genbank Accession No. NM_000572 or the like and which may be isolated or produced by publicly known methods. The "IL-10" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-12" is a material of which the human amino acid sequence and base sequence are published under the Genbank Accession No. NM_002187, NM_000882 or the like and which may be isolated or produced by publicly known methods. The "IL-12" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-13" is a material of which the human amino acid sequence and base sequence are published under the Genbank Accession No. NM_002188 or the like and which may be isolated or produced by publicly known methods. The "IL-13" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-15" is a material of which the human amino acid sequence and base sequence are published under the Genbank Accession No. NM_000585, NM_172174, NM_172175 or the like and which may be isolated or produced by publicly known methods. The "IL-15" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-17" is a material of which the human amino acid sequence and base sequence are published under the Genbank Accession No. NM_002190 or the like and which may be isolated or produced by publicly known methods. The "IL-17" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-18" is a material of which the human amino acid sequence and base sequence are published under the Genbank Accession No. NM_001562 or the like and which may be isolated or produced by publicly known methods. The "IL-18" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids. In the present invention, IL-18 is preferably a mature form.

In the present invention, "Eotaxin" is a material of which the human amino acid sequence and base sequence are published under the Genbank Accession No. D49372 or the like and which may be isolated or produced by publicly known methods. The "Eotaxin" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "FGF basic" is a material of which the human amino acid sequence and base sequence are published under the Genbank Accession No. NM_002006 or the like and which may be isolated or produced by publicly known methods. The "FGF basic" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids. The "FGF basic" is also referred to as "FGF-2".

In the present invention, "G-CSF" is a material of which the human amino acid sequence and base sequence are published under the Genbank or the like and which may be isolated or produced by publicly known methods. The "G-CSF" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "GM-CSF" is a material of which the human amino acid sequence and base sequence are published under the Genbank Accession No. X03021, M10633 or the like and which may be isolated or produced by publicly known methods. The "GM-CSF" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IFN-γ" is a material of which the human amino acid sequence and base sequence are published under the Genbank Accession No. NM_000619 or the like and which may be isolated or produced by publicly known methods. The "IFN-γ" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IP-10" is a material of which the human amino acid sequence and base sequence are published by the Genbank and the like and which may be isolated or produced by publicly known methods. The "IP-10" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "MCP-1" is a material of which the human amino acid sequence and base sequence are published by the Genbank and the like and which may be isolated or produced by publicly known methods. The "MCP-1" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "MIP-1α" is a material of which the human amino acid sequence and base sequence are published by the Genbank and the like and which may be isolated or produced by publicly known methods. The "MIP-1α" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "MIP-1β" is a material of which the human amino acid sequence and base sequence are published by the Genbank and the like and which may be isolated or produced by publicly known methods. The "MIP-1β" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "PDGF-BB" is a material of which the human amino acid sequence and base sequence are published by the Genbank and the like and which may be isolated or produced by publicly known methods. The "PDGF-BB" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "RANTES" is a material of which the human amino acid sequence and base sequence are published by the Genbank and the like and which may be isolated or produced by publicly known methods. The "RANTES" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "TNF-α" is a material of which the human amino acid sequence and base sequence are published by the Genbank and the like and which may be isolated or produced by publicly known methods. The "TNF-α" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "VEGF" is a material of which the human amino acid sequence and base sequence are published by the Genbank and the like and which may be isolated or produced by publicly known methods. The "VEGF" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-3" is a material of which the human amino acid sequence and base sequence are published by the NCBI Accession No.: Hs.694 and the like and which may be isolated or produced by publicly known methods. The "IL-3" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-11" is a material of which the human amino acid sequence and base sequence are published by the Genbank Accession No.: NM_000641 and the like and which may be isolated or produced by publicly known methods. The "IL-11" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the invention, "IFN-α," is a material of which the human amino acid sequence and base sequence are published by the Genbank and the like and which may be isolated or produced by publicly known methods. The "IFN-α," also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "CSF-2" is a material of which the human amino acid sequence and base sequence are published by the Genbank and the like and which may be isolated or produced by publicly known methods. The "CSF-2" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "TGF-β" is a material of which the human amino acid sequence and base sequence are published by the NCBI Accession No.: Hs.645227 and the like and which may be isolated or produced by publicly known methods. The "FGF-β" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "neurotrophin 5" is a material of which the human amino acid sequence and base sequence are published by the NCBI Accession No.: Hs.534255 and the like and which may be isolated or produced by publicly known methods. The "neurotrophin 5" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "MCP-3" is a material of which the human amino acid sequence and base sequence are published by the Genbank Accession No.: X72309 and the like and which may be isolated or produced by publicly known methods. The "MCP-3" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "β-2-microglobulin" is a material of which the human amino acid sequence and base sequence are published by the NCBI Accession No.: Hs.534255 and the like and which may be isolated or produced by publicly known methods. The "β-2-microglobulin" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "angiotensin II" is a material of which the human amino acid sequence and base sequence are publicly known and which may be isolated or produced by publicly known methods. The "angiotensin II" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "CSF-3" is a material of which the human amino acid sequence and base sequence are published by the NCBI Accession No.: Hs.2333 and the like and which may be isolated or produced by publicly known methods. The "CSF-3" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "CXC chemokine ligand 1" is a material of which the human amino acid sequence and base sequence are published by Genbank and the like and which may be isolated or produced by publicly known methods. The "CXC chemokine ligand 1" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "CXC chemokine ligand 5" is a material of which the human amino acid sequence and base sequence are published by Genbank and the like and which may be isolated or produced by publicly known methods. The "CXC chemokine ligand 5" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "HGF" is a material of which the human amino acid sequence and base sequence are published by the NCBI Accession No.: Hs.396530 and the like and which may be isolated or produced by publicly known methods. The "HGF" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, factors constituting the indicating agent, such as cytokines and chemokines, are preferably placed in different containers. In the present invention, the indicating agent may contain any other component, as long as it is mainly composed of the factors such as cytokines and chemokines. Examples of other components include, but are not limited to, a solvent such as a buffer and a saline, a stabilizing agent, a bacteriostatic agent, and a preservative.

In the present invention, the indicating agent serves to indicate that the physiological state of an animal is accompanied by stress or fatigue, preferably fatigue caused by mental stress. In particular, the degree of fatigue can be readily and quantitatively determined using the above-described factors, for the indicator, in a biological sample from an animal, preferably in blood plasma, serum, saliva, or urine. For example, in the case of a mouse, the content of IL-18 in serum is 100 pg/ml or less before the application of mental stress, and it becomes 120 to 200 pg/ml 5 hours after the application of 1 hour stress and exceeds 1000 pg/ml after the application of 6 hour stress. The content of IL-18 increases as the stress load increases. Since the half-life of IL-18 is about 10 hours, the IL-18-containing indicating agent is preferably used as a fatigue indicator. In addition to IL-18, an increase in the amount of various factors as describe above in a living organism can indicate fatigue. Specifically, at least two factors constituting the indicating agent of the present invention are preferably provided in amounts for indicating a normal level to a weak fatigue level or a strong fatigue level such that a comparison with samples can be made.

The agent for testing stress or fatigue of the present invention characteristically contains at least two kinds of molecules selected from the group consisting of molecules specifically recognizing IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, respectively.

Examples of the molecules include antibodies, peptides (other than antibodies), nucleic acids, and low-molecular-weight compounds. Antibodies are preferred, because they are relatively easy to obtain or produce.

The "antibodies" include polyclonal antibodies, monoclonal antibodies, chimeric antibodies, single-stranded antibodies, and antigen-binding part of the above antibodies, such as F(ab')₂ or Fab' fragments or fragments produced with Fab expression libraries.

The "peptides (other than antibodies)" include protein components capable of specifically binding to the factors (specific proteins), and examples include cytokine-binding proteins, cytokine receptors, cytokine soluble receptors, or binding sites thereof.

The "nucleic acids" include any nucleic acids having any specific base sequence capable of binding to the factors (specific proteins), and examples include DNAs, RNAs or nucleic acid analogues.

The "low-molecular-weight compounds" include organic or inorganic low-molecular-weight compounds designed based on the three-dimensional structure and electrostatic properties of the peptide.

The antibodies may be produced by conventional methods (Current Protocol in Molecular Biology, Chapter 11.12 - 11.13(2000)). Specifically, polyclonal antibodies may be produced by a process including the steps of immunizing non-human animals such as rabbits with any of the factors, which is expressed with E. coli or the like and purified by conventional techniques, or with a synthetic oligopeptide, having part of the amino acid sequence of any of the factors, by conventional techniques, and obtaining polyclonal antibodies from the serum of the immunized animals by conventional techniques. Alternatively, monoclonal antibodies may be produced by a process including the steps of immunizing non-human animals such as mice with any of the factors, which is expressed with E. coli or the like and purified by conventional techniques, or with an oligopeptide having part of the amino acid sequence of any of the factors, preparing hybridoma cells by cell fusion between the resulting spleen cells and myeloma cells, and culturing the hybridoma cells (Current protocols in Molecular Biology edit. Ausubel et al. (1987) Publish. John Wiley and Sons. Section 11.4 - 11.11). Chimeric antibodies may also be produced based on, for example, the techniques described in Jikken Igaku (Experimental Medicine), Extra Edition, Vol. 6, No. 10, 1988 or Japanese Patent Publication (JP-B) No. 03-73280. F(ab')₂ or Fab' may be produced by treating immunoglobulins with a proteolytic enzyme such as pepsin or papain.

The test agent may contain the antibody in a free form, a labeled form or an immobilized form. The test agent of the present invention may also contain a carrier, which is generally contained in diagnostic agents. Examples of such a carrier include, but are not limited to, a preservative, a stabilizing agent, a buffer, a solvent such as water or a saline and the like.

The present invention provides a method for measuring stress or fatigue, including the step of measuring the amounts of factors in a biological sample with the test agent.

In the stress or fatigue measurement method, the amounts of the factors in a biological sample, preferably in blood plasma, serum, saliva, or urine, may be quantitatively measured with the molecules in the test agent, preferably with the antibodies in the test agent, by publicly known methods. Systems that allow simple and simultaneous measurement of a number of proteins preferably include liquid-phase protein array systems (such as Bio-Plex (trade name) Suspension Array System (manufactured by Bio-Rad Laboratories)) in which protein recognition sensor-carrying microbeads are used to perform a binding reaction in a liquid suspension of the microbeads. When such array systems are used, measurement is possible in a wide range of a few pg/ml to several tens ng/ml.

The measured amount of each factor in the biological sample may be compared with the level of the stress or fatigue indicating agent so that the degree of stress or fatigue in the animal can be objectively and qualitatively or quantitatively evaluated.

The animal is preferably a vertebrate including human and particularly preferably a domestic or companion animal such as cattle, horse, pig, sheep, goat, chicken, dog, cat and the like. The stress or fatigue measurement method of the invention may be applied to a domestic or companion animal. In the field of livestock or pet business where artificial rearing tends to cause stress, therefore, the state of stress or fatigue in an animal can be objectively monitored, which is advantageous for grasping and well controlling the health of the animal.

In the test agent of the invention, the molecules, preferably antibodies are preferably placed in different containers. The molecules placed in different containers may form a test kit for determining the intensity of mental conditions or disorders as described later. The test kit may include any other reagent such as a buffer for dilution of the reagent or the biological sample, a fluorescent dye, a reaction vessel, a positive control, a negative control, and test protocol instructions. The intensity of mental conditions or disorders can be easily measured using the kit of the invention.

The present invention can provide at least two kinds of weighted factors selected from the group consisting of IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTS, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, as an indicating agent for evaluating mental conditions selected from the group consisting of mental fatigue, physical fatigue, stress, blue mood, comfortable mood, uncomfortable mood, dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk.

Here, depression-like mental conditions or schizophrenia-like mental conditions refer to mental conditions that may appear to be depression or schizophrenia but are not actually associated with the presence or development of such mental disorders. Depression- or schizophrenia-like conditions also include mental disorder-like mental conditions such as mood disorders, obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, and suicide attempt.

The present invention can provide at least two kinds of weighted factors selected from the group consisting of IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β PDGF-BB, RANTS, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, as an indicating agent for evaluating intensity of mental disorders selected from the group consisting of mental fatigue, stress, depression, depressive state, mood disorders, schizophrenia, obsessive-compulsive disorder, panic disorder, anxiety disorder, phobia, anthropophobia, social phobia, excessive tension, maladjustment to job or learning, maladjustment to job or learning, suicide feeling, personality disorder, alcoholic psychoses, insomnia, diurnal rhythm disorder, psychoneurosis, dementia (Alzheimer type senile dementia, Alzheimer disease, Pick disease), central neurodegenerative disease (OPCA (olivopontocerebellar atrophy), Parkinson disease, diffuse Lewy body disease) and suicide attempt.

All the indicating agents make it possible to evaluate not only conditions, disorders or development of diseases but also premorbid conditions (where physical examinations cannot reveal explicit diseases, but some pathological condition or any pathological sign exists so that the risk of development can be significantly predicted).

In the present invention, "stress" means various biological responses caused by the application of a chemical, physical, mental, verbal, or laborious temporary load to the mental or physical condition of a living organism. An index of specific stress caused by a specific load may be provided depending on how to weight the factors. Specifically, each factor may be multiplied by a weighting coefficient obtained by distribution calculation so that an index of stress for a mental condition or disorder can be provided.

In the present invention, "fatigue" includes physical fatigue and mental fatigue. Any of a physical fatigue-weighted index and a mental fatigue-weighted index may be provided depending on how to weight the factors. Specifically, each factor may be multiplied by a weighting coefficient obtained by distribution calculation so that an index of a mental disorder based on mental fatigue or an index of a mental condition caused by physical fatigue and mental fatigue can be provided.

In the present invention, the "blue mood" is as defined in DSM-IV.

As used herein, the term "depression" refers to the contents defined in DSM-IV and a group of diseases called "major depression" in the field of psychiatry.

As used herein, the term "dysthymia," refers to ups and downs of mood.

In the present invention, the "mood disorders" are as defined in DSM-IV.

In the present invention, the "schizophrenia" is as defined in DSM-IV.

In the present invention, the "compulsive" is as defined in DSM-IV.

In the present invention, the "obsessive-compulsive disorder" is as defined in DSM-IV.

In the present invention, the "panic" is as defined in DSM-IV.

In the present invention, the "panic disorder" is as defined in DSM-IV.

In the present invention, the "anxiety" is as defined in DSM-IV.

In the present invention, the "anxiety disorder" is as defined in DSM-IV.

In the present invention, the "phobia" is as defined in DSM-IV.

In the present invention, the "anthropophobia" is as defined in DSM-IV.

In the present invention, the "social phobia" is as defined in DSM-IV.

As used herein, the term "tension" refers to responses to a chemical, physical, mental, verbal, or laborious load that are mainly characterized by psychentonia or sthenia (response) of parasympathetic nervous system.

In the present invention, the "excessive tension" is as defined in DSM-IV.

As used herein, the term "labor intensity" refers to the degree of fatigue that may be determined by Kraepelin test, fatigue investigation, or the like after labor.

In the present invention, the "maladjustment to job" is as defined in DSM-IV.

In the present invention, the "learning intensity" refers to the degree of fatigue that may be determined by Kraepelin test, fatigue investigation, or the like after learning.

In the present invention, the "maladjustment to learning" is as defined in DSM-IV.

In the present invention, the "suicide risk" is as defined in DSM-IV.

In the present invention, the "suicide feeling" is as defined in DSM-IV.

In the present invention, the "suicide attempt" is as defined in DSM-IV.

In the present invention, the "personality disorder" is as defined in DSM-IV.

In the present invention, the "alcoholic psychoses" is as defined in DSM-IV.

In the present invention, the "insomnia" is as defined in DSM-IV.

In the present invention, the "diurnal rhythm disorder" is as defined in DSM-IV.

In the present invention, the "psychoneurosis" is as defined in DSM-IV.

In the present invention, the "comfortable mood" is as defined in DSM-IV.

In the present invention, the "uncomfortable mood" is as defined in DSM-IV.

In the present invention, the "depressive state" is as defined in DSM-IV.

In the present invention, the "dementia" is as defined in DSM-IV.

In the present invention, the "Alzheimer type senile dementia" is as defined in DSM-IV.

In the present invention, the "Alzheimer disease" is as defined in DSM-IV.

In the present invention, the "Pick disease" is as defined in DSM-IV.

In the present invention, the "central neurodegenerative disease" is as defined in DSM-IV.

In the present invention, the "OPCA (olivopontocerebellar atrophy)" is as defined in DSM-IV.

In the present invention, the "Parkinson disease" is as defined in DSM-IV.

In the present invention, the "diffuse Lewy body disease" is as defined in DSM-IV.

Mental disorders other than those specified above also fall within the scope of the invention, as long as they are defined in DSM-IV. If DSM-IV is revised, the revised version will also fall within the scope of the invention.

In the present invention, the indicating agent of the intensity of mental conditions or disorders includes at least two kinds of factors that are selected from the group described above and are each weighted. In order to make the indicating agent more specific to the intensity of each mental condition or disorder, 3 to 41 kinds of factors, preferably 3 to 28 kinds, more preferably 5 to 20 kinds, even more preferably 8 to 12 kinds should be selected.

In the present invention, factors constituting the indicating agent of the intensity of mental conditions or disorders, such as cytokines and chemokines, are preferably placed in different containers. In the present invention, the indicating agent of mental conditions may contain any other component, as long as it is mainly composed of the factors such as cytokines and chemokines. Examples of other components include, but are not limited to, a solvent such as a buffer and a saline, a stabilizing agent, a bacteriostatic agent, a preservative and the like.

The indicating agent of the intensity of a mental condition or disorder according to the present invention may serve as an objective indicator in the field of animal mental health. In particular, factors in an animal biological sample, preferably in blood plasma, serum, saliva, or urine, may be each weighted to constitute the indicating agent so that various mental conditions can be readily and quantitatively determined.

In the present invention, the indicating agent of fatigue or the indicating agent of the intensity of a mental condition or disorder may comprise a combination of numerical values of weighted factors, namely a database. When the indicating agent is a database, various mental conditions can be readily and quantitatively determined for each item from the values measured with the test agent of the invention.

The agent for testing intensity of mental conditions or mental disorders of the present invention characteristically contains at least two kinds of molecule selected from the group consisting of molecules specifically recognizing the aforementioned factors (IL-1β, IL-1ra, IL-2, IL-3, IL-4, IL-5, IL-6, IL-7, IL-8, IL-9, IL-10, IL-11, IL-12, IL-13, IL-15, IL-17, IL-18, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, FGF-β, neurotrophin 5, MCP-3, β-2-microglobulin, angiotensin II, CSF-3, CXC chemokine ligand 1, CXC chemokine ligand 5 and HGF, respectively).

The molecules and other components contained in the agent for testing mental conditions or disorders of the present invention may be the same as those contained in the agent for testing stress or fatigue.

The present invention also provides a method for measuring the intensity of a mental condition or disorder, including the steps of: measuring the amounts of factors in a biological sample with the agent for testing the intensity of a mental condition or disorder; and proportionally weighting each of the amounts obtained in the measuring step and comparing the weighted amounts with the indicating agent of the intensity of the mental condition or disorder.

In the method for measuring the intensity of a mental condition or disorder, the concentrations of the factors in a biological sample, preferably in blood plasma, serum, saliva, or urine, may be measured with the molecules, preferably antibodies, in the agent for testing the intensity of the mental condition or disorder, by publicly known methods. For example, the system that allows simple and simultaneous measurement of a number of proteins is preferably Bio-Plex Suspension Array System (manufactured by Bio-Rad Laboratories).

The value obtained by multiplying the measured amount of each factor in the biological sample by a coefficient is compared with the indicating agent of the mental condition so that the mental condition of an animal can be objectively and quantitatively evaluated.

The animal is preferably a vertebrate including human and particularly preferably a domestic or companion animal such as cattle, horse, pig, sheep, goat, chicken, dog, cat and the like. The method for measuring the intensity of a mental condition or disorder of the present invention may be applied to a domestic or companion animal. In the field of livestock or pet business where artificial rearing tends to cause stress, therefore, the intensity of the mental condition or disorder of an animal can be objectively monitored, which is advantageous for grasping the mental health of the animal.

The kit for testing the intensity of a mental condition or disorder of the present invention includes the molecules, preferably at least two kinds of antibodies, placed in different compartments. The test kit may include any other reagent such as a buffer for dilution of the reagent or the biological sample, a fluorescent dye, a reaction vessel, a positive control, a negative control, test protocol instructions and the like. Mental conditions can be easily measured using the test kit of the present invention.

According to the present invention, the levels of the factors in various mental conditions or disorders can be analyzed with high throughput. Individual differences can also be easily found based on the findings obtained by the present invention. Therefore, the present invention may be applied to discover new genes based on individual differences in the response of cytokine level to stress or may be applied to screening for stress resistance. The present invention may also be used to determine the anti-stress effect of anti-stress drugs. The present invention can provide the following advantages:
1. A set uniquely developed for stress indication may be used for measurement;
2. All the factors can be measured using 50 µl of a blood plasma or serum sample;
3. The entire process can be completed in about 6 hours; and
4. All of the resulting data may be compiled into a database, which can be easily statistically processed.

When the advantages are exploited for measurement of stress and so on, stress or other mental conditions can be grasped in a significantly inexpensive and speedy manner, as compared with conventional methods in which data are collected through an interview by a physician or psychologist and with a questionnaire and then compiled over several days to be evaluated (so that the result can vary between the facilities).

### Examples

The invention is more specifically described below with some examples and so on which are not intended to limit the scope of the invention. In the examples described below, before volunteer subjects underwent different tests and blood collection, approval of the ethical committee of the facility and then an informed consent were obtained in advance.

### Example 1: Scales of Mental Condition and Fatigue Condition

Psychological tests by questionnaire methods (SDS, MAS, GHQ, STAI, and CMI), a subjective symptom survey (prepared by Institute of Occupational Health), and surveys with a social phobia scale, an anthropophobia scale and a uniquely developed life situation questionnaire were performed on 402 volunteer subjects, so that it was confirmed that they were healthy. After the health check, 1 ml of blood was collected from each subject on a holiday morning and at the same time point after a night shift, respectively, and placed in an anticoagulant-containing blood collection spitz. Plasma was then separated from the blood under cooling at 4°C. The blood plasma was frozen and stored or stored on ice, and then the cytokines shown separately were simultaneously measured with a microbead protein array system (Bio-Plex Suspension Array System manufactured by Bio-Rad Laboratories and modified by ourselves). The cytokines were also measured by a conventional ELISA method, and the concentration of each cytokine in the blood was determined.

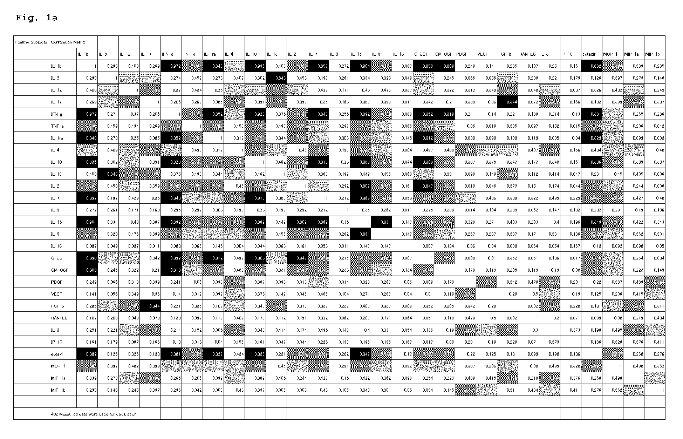

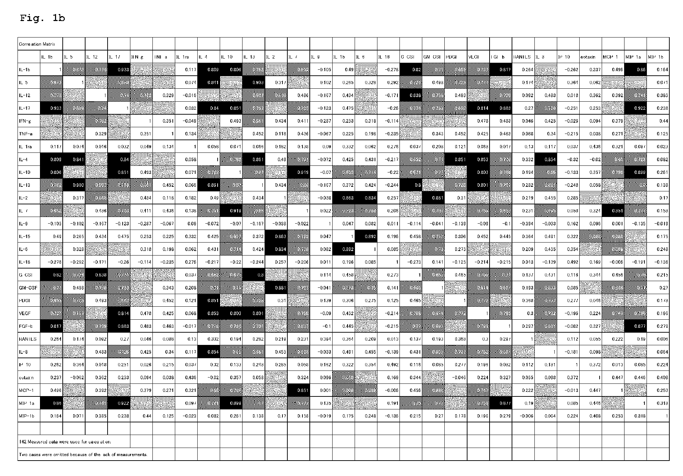

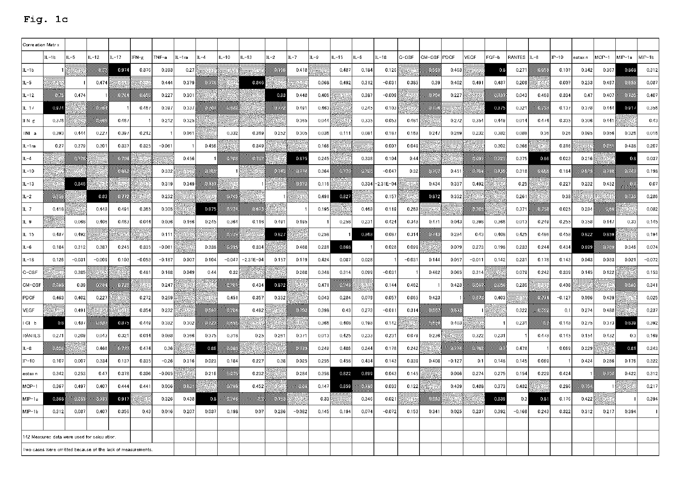

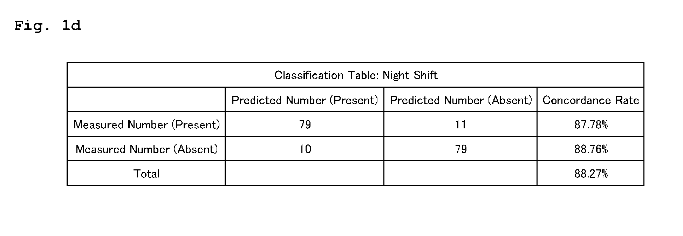

An example of the result is shown in Fig. 1a. The result was obtained by a process including determining the concentration of each cytokine in the blood, then calculating a correlation coefficient for each cytokine concentration in blood, and coloring high correlation values (with correlation coefficients of 0.50 to 0.6499 in light gray, 0.65 to 0.7999 in dark gray, 0.8 to 0.9999 in black; concerning the relative risk of correlation, p < 0.001 when the correlation coefficient was 0.50). The result shows that some cytokines have high correlation in the healthy subjects. Then, 142 subjects who were healthy but frequently work night shifts were examined as to whether the cytokine correlation could reflect the presence or absence of a night shift and was useful to discriminate between before and after a night shift. It is apparent that the correlation with the concentration of each cytokine in blood has changed between Fig. 1b (before a night shift) and Fig. 1c (after a night shift). It is recognized that the concentrations at the same time point after the night shift significantly deviated from normal values. Based on the cytokine correlation, a logistic regression analysis was performed on the data from 90 people randomly sampled from the subjects to determine whether discrimination between before and after a night shift was possible according to the present invention. The result is shown in Fig. 1d. In this example, the accuracy was 88%. Each cytokine value was multiplied by a certain coefficient for each of the items of depression, anxiety, fear, and stress in the psychological test, so that scores were produced. The resulting scores agreed with the results of interview by a psychiatrist more clearly than the psychological test, and therefore, it was possible to detect a tendency to depression, anxiety, or stress. This shows that different mental conditions (such as depression, excitement, tension, anxiety and the like) can be grasped and evaluated by the measurement method of the present invention.

### Example 2: Scale of Mental Fatigue or Physical Fatigue and Scale of Stress

Twenty-eight to 30 healthy volunteers underwent a Kraepelin test in which they continued simple calculation as a load on mental fatigue for 3 hours or a test in which an aerobic treadmill exercise to increase the 1-minute pulse rate to 180 was applied as a load on physical fatigue for 3 hours. Before and after each test, blood was collected and subjected to the measurement of cytokines in the same way as in Example 1. The cytokines were also measured in the same way 24 hours after the start of the load (21 hours after the end of the load). The result was obtained by calculating a correlation coefficient for each cytokine concentration in blood and coloring high correlation values.
The result showed that the correlation with the total cytokines changed before and after each test and during the recovery period. A comparison between the calculation for the mental load and the calculation for the treadmill exercise as the physical load showed that a certain combination of cytokines made a difference in the strength of the correlation and that fatigue and stress conditions can be grasped and evaluated by the measurement method of the present invention.
Based on the cytokine correlation, a logistic regression analysis was performed on the data from all the subjects to determine whether it was possible to discriminate between the Kraepelin test as a mental load and the treadmill exercise as a physical load. The result is shown in the drawings. The result showed that: it was possible to distinguish between a long loading time and a short loading time with respect to both the Kraepelin test and the treadmill test (in the example, the accuracy was 100% for the Kraepelin test and 81% for the treadmill test); it was also possible to show a difference in the recovery time between the subjects (in the example, the accuracy was 81% for the Kraepelin test and 100% for the treadmill test); and it was also possible to determine which load (Kraepelin load or treadmill load) was applied (in the example, the accuracy was 100%). This suggests that according to the present invention, not only the presence or absence of fatigue but also the type and level of fatigue can be classified, identified, or evaluated. It is also suggested that the improvement effect or effect of factors such as drugs, food products and living habits on mental fatigue or physical fatigue can be clearly shown using the accuracies shown in the classification tables obtained according to the present invention and taking into account that the recovery degree under the standard conditions may depend on drugs, food products, living habits and so on.

### Example 3: Scales for Diagnosis and Evaluation of Mental Disorders

Psychological tests by questionnaire methods (SDS, MAS, GHQ, STAI, SCID, and CMI), a subjective symptom survey (prepared by Institute of Occupational Health), and surveys with a social phobia scale, an anthropophobia scale and a uniquely developed life situation questionnaire were performed, as much as possible, on 160 volunteers diagnosed as having mental disorders according DSM-IV international diagnostic criteria.
Concurrently with the surveys, 1 ml of blood was collected from each volunteer and placed in an anticoagulant-containing blood collection spitz. Plasma was then separated from the blood under cooling at 4°C. The blood plasma was frozen and stored or stored on ice, and then the cytokines shown separately were simultaneously measured with a microbead protein array system (Bio-Plex Suspension Array System manufactured by Bio-Rad Laboratories and modified by ourselves). The cytokines were also measured by a conventional ELISA method, and the concentration of each cytokine in the blood was determined. On the other hand, all the volunteers each had a psychiatrically structured interview so that their psychological tendency and conditions were grasped.
Each cytokine value was multiplied by a certain coefficient to produce scores. The resulting scores agreed with the results of interview by a psychiatrist more clearly than the psychological test, and therefore, it was possible to detect a tendency to mental conditions (depression, anxiety, stress, tension, and excitement). Although the psychological test scores are not disclosed for maintenance of the confidentiality of personal information of each patient, Fig. 3 shows cytokine correlation coefficients for patients with depression, and Fig. 4 shows cytokine correlation coefficients for patients with schizophrenia. The results were obtained by calculating a correlation coefficient for each cytokine concentration in blood and coloring high correlation values. It is apparent that the results each differ from the correlation coefficient table of the healthy subjects shown in Fig. 1. A logistic regression analysis was performed to determine whether it was possible to correctly diagnose depression according to the present invention. As a result, the accuracy was 91%.
A logistic regression analysis was also performed to determine whether it was possible to correctly diagnose schizophrenia according to the present invention. As a result, the accuracy was 96%.
The results show that different mental disorders (such as depression, schizophrenia, excitement, tension, anxiety and the like) can be grasped and evaluated by the cytokine measurement method according to the present invention. The results also show that psychiatric diagnosis or therapy validation, which would conventionally significantly depend on empirical determination and tend to be ambiguous, can be definitely made using the cytokine measurement and that the cytokine measurement would be useful for evaluation of therapeutic regimen efficacy. Mental conditions are often not externalized even though they are in a pathological state, and this adds to the problem in the current society. It has been found, however, that based on the above correlation found according to the present invention, diagnosis or assessment of depression and schizophrenia, two major diseases in the field of psychiatry, can be satisfactorily performed according to the present invention.

Predictions about dementia (senile dementia of Alzheimer type, Alzheimer disease and Pick disease) and central neurodegenerative diseases (OPCA, Parkinson disease, and diffuse Lewy body disease), if possible, should greatly contribute to the QOL of patients. According to the present invention, discrimination between depression, mild dementia and central neurodegenerative disease is possible at a stage where dementia or central neurodegenerative disease is not manifested. Therefore, it is expected that grouping of mild dementia or mild degenerative disease as proposed in the field of psychiatry can be achieved.

According to the present invention, the use of the correlation found between mental condition, fatigue or stress level and levels of the factors in blood, urine or saliva allows simple, inexpensive and objective evaluation of the risk or degree of stress, fatigue or dysthymia (particularly blues). The allocation of the score to each of the selected cytokines may be modified so that stress, fatigue and dysthymia (particularly blues) can each be rated on an optimum scale in a single measurement procedure, and this is widely applicable.
Therefore, stress, fatigue or dysthymia, (blues) can be objectively evaluated and their degree can be grasped. Since cytokines are causative agents or exacerbation factors in various diseases, care and preventive measures can be provided from the standpoint of the integration of mind and body.

### Industrial Applicability

According to the invention, the intensity of external loads or the effect of the loads can be evaluated or predicted, when temporary biological reactions occur. Specifically, external load-induced fatigue and relief from the fatigue can be evaluated, so that the individual's tendency to become fatigued or the individual's resilience can be evaluated. In addition, the degree of the effect of a certain factor on the individual's tendency to become fatigued or the individual's resilience can also be evaluated. Therefore, the application of the present invention to the field of occupational health may lead to prevention of occupational accidents. In addition, the present invention can contribute to the development of stress-relieving pharmaceuticals, food products, daily necessities, designs, images, sounds, buildings, residential environments, and so on.

According to the present invention, evaluation of disease states, recognition of potential pathological conditions, and detection of diseases before the manifestation of subjective symptoms are possible in a living organism with constant pathological conditions. Therefore, the present invention allows early detection and early treatment of pathological conditions, evaluation of treatment effects, determination of whether therapies are effective or ineffective, and unified detection and management of many pathological conditions (such as skin disorders caused by ultraviolet rays and so on, atopic dermatitis, psoriasis, acne vulgaris, pemphigus, ichthyosis, optic hyperesthesia, burns including inflammatory skin changes as pathological conditions, radiation dermatosis, Alzheimer disease, senile dementia of Alzheimer type, diffuse Lewy body disease, Pick disease, Binswanger disease, Parkinson disease, Parkinson syndrome, cerebral ischemia, cerebral ischemia/reperfusion injury, carbon monoxide poisoning,

## Claims

1. An agent for testing mental conditions by a logistic regression analysis, which mental conditions are selected from the group consisting of mental fatigue, physical fatigue, stress, blue mood, comfortable mood, uncomfortable mood, dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk, which test agent comprises at least three kinds of molecules selected from the group consisting of antibodie specifically recognizing Eotaxin, FGF basic, G-CSF, GM-CSF, IP-10, MCP-1, MIP-1α, MIP-1β, PDGF-BB, PANTIES, CSF-2, neurotrophin 5, MCP-3, β-2-microglobulin, angiotensin II, CSF-3, HGF, VEGF, FGF-β, IL-3, IL-5, IL-7, IL-9, IL-10, IL-11, IL-12, IL-13, IL-15, IL-17, IFN-γ, IFN-α, CXC chemokine ligand 5, IL-4, IL-8, IL-2, TNF-α, CXC chemokine ligand 1, IL-1β and IL-1ra, respectively.

2. An agent for testing intensity of mental disorders by a logistic regression analysis, which mental disorders are selected from the group consisting of mental fatigue, stress, depression, depression state, mood disorders, schizophrenia, obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt, which test agent comprises at least three kinds of molecules selected from the group consisting of antibodies specifically recognizing Eotaxin, FGF basic, G-CSF, GM-CSF, IP-10, MCP-1, MIP-1α, MIP-1β, PDGF-BB, RANTES, CSF-2, neurotrophin 5, MCP-3, β-2-microglobulin, angiotensin II, CSF-3, HGF, VEGF, TGF-β, IL-3, IL-5, IL-7, IL-9, IL-10, IL-11, IL-12, IL-13, IL-15, IL-17, IFN-γ, IFN-α, CXC chemokine ligand 5, IL-4, IL-8, IL-2, TNF-α, CXC chemokine ligand 1, IL-1β and IL-1ra, respectively.

3. A method of measuring mental conditions selected from the group consisting of mental fatigue, physical fatigue, stress, blue mood, comfortable mood, uncomfortable mood, dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk, which comprises a step of measuring the level of the factors in a biological sample using the test agent of claim 1, and
a step of comparing the amount obtained in the aforementioned measurement step with that of an indicating agent by proportional weighting,
which indicating agent is used for evaluating mental conditions selected from the group consisting of mental fatigue, physical fatigue, stress, blue mood, comfortable mood, uncomfortable mood, dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk, and comprises at least three kinds of factors selected from the group consisting of Eotaxin, FGF basic, G-CSF, GM-CSF, IP-10, MCP-1, MIP-1α, MIP-1β, PDGF-BB, RANTES, CSF-2, neurotrophin 5, MCP-3, β-2-microglobulin, angiotensin II, CSF-3, HGF, VEGF, FGF-β, IL-3, IL-5, IL-7, IL-9, IL-10, IL-11, IL-12, IL-13, IL-15, IL-17, IFN-γ, IFN-α, CXC chemokine ligand 5, IL-4, IL-8, IL-2, TNF-α, CXC chemokine ligand 1, IL-1βand IL-1ra at a weighted composition ratio.

4. A method of measuring the intensity of mental disorders selected from the group consisting of mental fatigue, stress, depression, depression state, mood disorders, schizophrenia, obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt, which comprises a step of measuring the level of the factors in a biological sample using the test agent of claim 2, and
a step of comparing the amount obtained in the aforementioned measurement step with that of an indicating agent by proportional weighting,
which indicating agent is used for evaluating intensity of mental disorders selected from the group consisting of mental fatigue, stress, depression, depression state, mood disorders, schizophrenia, obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt, and comprises at least three kinds of factors selected from the group consisting of Eotaxin, FGF basic, G-CSF, GM-CSF, IP-10, MCP-1, MIP-1α, MIP-1β, PDGF-BB, RANTS, CSF-2, neurotrophin 5, MCP-3, β-2-microglobulin, angiotensin II, CSF-3, HGF, VEGF, TGF-β, IL-3, IL-5, IL-7, IL-9, IL-10, IL-11, IL-12, IL-13, IL-15, IL-17, IFN-γ, IFN-α, CXC chemokine ligand 5, IL-4, IL-8, IL-2, TNF-α, CXC chemokine ligand 1, IL-1β and IL-1ra at a weighted composition ratio.

5. The method of claim 3 or 4, wherein the aforementioned biological sample is plasma, serum, saliva or urine.

6. A kit for testing mental conditions by a logistic regression analysis, which mental conditions are selected from the group consisting of mental fatigue, physical fatigue, stress, blue mood, comfortable mood, uncomfortable mood, dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk, comprising, in separate compartments, at least three kinds of molecules selected from the group consisting of antibodies specifically recognizing Eotaxin, FGF basic, G-CSF, GM-CSF, IP-10, MCP-1, MIP-1α, MIP-1β, PDGF-BB, RANTES, CSF-2, neurotrophin 5, MCP-3, β-2-microglobulin, angiotensin II, CSF-3, HGF, VEGF, FGF-β, IL-3, IL-5, IL-7, IL-9, IL-10, IL-11, IL-12, IL-13, IL-15, IL-17, IFN-γ, IFN-α, CXC chemokine ligand 5, IL-4, IL-8, IL-2, TNF-a, CXC chemokine ligand 1, IL-1β and IL-1ra, respectively.

7. A kit for testing the intensity of mental disorders by a logistic regression analysis, which mental disorders are selected from the group consisting of mental fatigue, stress, depression, depression state, mood disorders, schizophrenia, obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt, comprising, in separate compartments, at least three kinds of molecules selected from the group consisting of antibodies specifically recognizing Eotaxin, FGF basic, G-CSF, GM-CSF, IP-10, MCP-1, MIP-1α, MIP-1β, PDGF-BB, RANTES, CSF-2, neurotrophin 5, MCP-3, β-2-microglobulin, angiotensin II, CSF-3, HGF, VEGF, FGF-β, IL-3, IL-5, IL-7, IL-9, IL-10, IL-11, IL-12, IL-13, IL-15, IL-17, IFN-γ, IFN-α, CXC chemokine ligand 5, IL-4, IL-8, IL-2, TNF-α, CXC chemokine ligand 1, IL-1β and IL-1ra, respectively.

8. An indicating agent for evaluating mental conditions selected from the group consisting of mental fatigue, physical fatigue, stress, blue mood, comfortable mood, uncomfortable mood, dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk, which comprises at least three kinds of factors selected from the group consisting of Eotaxin, FGF basic, G-CSF, GM-CSF, IP-10, MCP-1, MIP-1α, MIP-1β, PDGF-BB, RANTES, CSF-2, neurotrophin 5, MCP-3, β-2-microglobulin, angiotensin II, CSF-3, HGF, VEGF, TGF-β, IL-3, IL-5, IL-7, IL-9, IL-10, IL-11, IL-12, IL-13, IL-15, IL-17, IFN-γ, IFN-α, CXC chemokine ligand 5, IL-4, IL-8, IL-2, TNF-α, CXC chemokine ligand 1, IL-1β and IL-1ra at a weighted composition ratio.

9. An indicating agent for evaluating intensity of mental disorders selected from the group consisting of mental fatigue, stress, depression, depression state, mood disorders, schizophrenia, obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt, which comprises at least three kinds of factors selected from the group consisting of Eotaxin, FGF basic, G-CSF, GM-CSF, IP-10, MCP-1, MIP-1α, MIP-1β, PDGF-BB, RANTS, CSF-2, neurotrophin 5, MCP-3, β-2-microglobulin, angiotensin II, CSF-3, HGF, VEGF, FGF-β, IL-3, IL-5, IL-7, IL-9, IL-10, IL-11, IL-12, IL-13, IL-15, IL-17, IFN-γ, IFN-α, CXC chemokine ligand 5, IL-4, IL-8, IL-2, TNF-α, CXC chemokine ligand 1, IL-1β and IL-1ra at a weighted composition ratio.

10. The agent of claim 1, wherein the mental conditions are selected from the group consisting of mental fatigue, physical fatigue, stress, blue mood, comfortable mood and uncomfortable mood; which are related to dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk.

11. The agent of claim 2, wherein the mental disorderes are selected from the group consisting of mental fatigue, stress, depression, depression state, mood disorders and schizophrenia; which are related to obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt.

12. The method of claim 3 or 5, wherein the mental conditions are selected from the group consisting of mental fatigue, physical fatigue, stress, blue mood, comfortable mood and uncomfortable mood; which are related to dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk, and
Wherein the indicating agent is used for evaluating mental conditions selected from the group consisting of mental fatigue, physical fatigue, stress, blue mood, comfortable mood, and uncomfortable mood; which are related to dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk.

13. The method of claim 4 or 5, wherein the mental disorderes are selected from the group consisting of mental fatigue, stress, depression, depression state, mood disorders and schizophrenia; which are related to obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt, and wherein the indicating agent is used for evaluating intensity of mental disorders selected from the group consisting of mental fatigue, stress, depression, depression state, mood disorders and schizophrenia; which are related to obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt.

14. The kit of claim 6, wherein mental conditions are selected from the group consisting of mental fatigue, physical fatigue, stress, blue mood, comfortable mood, and uncomfortable mood; which are related to dysthymia, compulsive, panic, anxiety, phobia, anthropophobia, social phobia, tension, working intensity, learning intensity, depression, schizophrenia, mental conditions similar to depression, mental conditions similar to schizophrenia and suicide risk.

15. The kit of claim 7, wherein the mental disorders are selected from the group consisting of mental fatigue, stress, depression, depression state, mood disorders, and schizophrenia; which are related to obsessive-compulsive disorder, panic disorder, anxiety disorder, phobia, anthropophobia, social phobia, excessive tension, maladjustment to job or learning, suicide feeling, personality disorder, alcoholic psychoses, insomnia, diurnal rhythm disorder, psychoneurosis, dementia, central neurodegenerative disease and suicide attempt.
